(19) Europäisches Patentamt
European Patent Office
Office européen des brevets

(11) Veröffentlichungsnummer : **0 410 243 B1**

# (12) EUROPÄISCHE PATENTSCHRIFT

(45) Veröffentlichungstag der Patentschrift : **18.08.93 Patentblatt 93/33**

(51) Int. Cl.[5] : **C07C 209/18,** C07C 211/48

(21) Anmeldenummer : **90113515.2**

(22) Anmeldetag : **14.07.90**

## (54) Verfahren zur Herstellung von N-alkylierten Anilinen.

(30) Priorität : **28.07.89 DE 3924999**

(43) Veröffentlichungstag der Anmeldung : **30.01.91 Patentblatt 91/05**

(45) Bekanntmachung des Hinweises auf die Patenterteilung : **18.08.93 Patentblatt 93/33**

(84) Benannte Vertragsstaaten : **BE DE FR GB IT**

(56) Entgegenhaltungen :
**DE-A- 2 335 906**
**GB-A- 577 901**
**GB-A- 915 186**
**US-A- 4 613 705**
**HOUBEN-WEYL, Methoden der organischen Chemie, Band X1/1, Stickstoffverbindungen II, 1957, Seiten 112-119, Georg Thieme Verlag, Stuttgart, DE**

(73) Patentinhaber : **BAYER AG**
**D-51368 Leverkusen (DE)**

(72) Erfinder : **Wimmer, Peter, Dr.**
**Bethelstrasse 10**
**D-4150 Krefeld (DE)**
Erfinder : **Buysch, Hans-Josef, Dr.**
**Brandenburgerstrasse 28**
**D-4150 Krefeld (DE)**
Erfinder : **Puppe, Lothar, Dr.**
**Am Weiher 10a**
**D-5093 Burscheid (DE)**
Erfinder : **Froehlich, Christian, Dr.**
**Doerperhofstrasse 14**
**D-4150 Krefeld (DE)**

EP 0 410 243 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von N-alkylierten Anilinen durch Umsetzung von nicht oder nur einfach alkylierten Anilinen mit niederen Alkoholen bzw. den zugehörigen Ethern bei erhöhter Temperatur in der Gasphase in Gegenwart eines sauren Aluminiumoxidkatalysators.

Alkylierte Aniline sind wichtige industrielle Zwischenprodukte zur Herstellung von Farbstoffen, Stabilisatoren, Urethanen, Harnstoffen, Pharmaka und Pflanzenschutzmitteln.

Es ist bekannt, N-alkylierte Aniline durch Umsetzung von Anilinen mit Alkoholen sowohl in der Flüssig- als auch in der Gasphase in Gegenwart saurer Katalysatoren herzustellen. Im Flüssigphasenverfahren wird die Reaktion in Gegenwart eines flüssigen Katalysators, wie beispielsweise Schwefelsäure, Salzsäure oder Phosphortrichlorid, durchgeführt. Diese Verfahren erfordern eine Arbeitsweise unter Druck. Wegen der stark korrosiven Wirkung der sauren Katalysatoren muß in besonders korrosionsfesten Autoklaven gearbeitet werden. Dennoch ist Korrosion nicht zu vermeiden. Darüber hinaus erfordert die Abtrennung und Entsorgung des Katalysators einen zusätzlichen Aufwand.

Gasphasenverfahren werden dagegen drucklos durchgeführt. So wird nach dem in der DE-AS 10 31 796 beschriebenen Verfahren ein Gemisch von Anilin- und Alkoholdampf bei Normaldruck durch heiße Phosphorsäure geleitet. Neben Korrosionsproblemen hat dieses Verfahren den Nachteil, daß die relativ große Menge Phosphorsäure im Laufe der Zeit als Katalysator unbrauchbar wird und durch frische ersetzt werden muß. Der verbrauchte Katalysator muß einer fachgerechten Entsorgung zugeführt werden.

Es ist weiter bekannt, die Alkylierung von aromatischen Aminen mit Alkoholen an übergangsmetallhaltigen Katalysatoren durchzuführen. Die US 4.613.705 beschreibt beispielsweise einen Vanadium/Zinnoxid-Kontakt. Der Umsatz an Anilin an diesem Katalysator ist jedoch unbefriedigend und es findet sich ein hoher Anteil unerwünschter kernalkylierter Produkte.

Aus Houben-Weyl, Methoden der organischen Chemie, Band XI/1 (1957), S. 116 ist bekannt, daß auch Aluminiumoxide oder Silikate geeignete Katalysatoren für die Alkylierung aromatischer Amine sind. Wie weiter beschrieben ist, haben diese Katalysatoren jedoch den Nachteil, daß ihre Aktivität zu rasch abnimmt und die Lebensdauer für technische Zwecke nicht ausreichend ist.

Die DE-AS 23 35 906 lehrt, daß man Katalysatoren mit hoher Standzeit erhält, wenn man Kieselsäure mit einem Gehalt von 0,1 bis 20 Gew.-% Phosphorsäure versetzt. Um auch hier eine rasche Desaktivierung zu vermeiden und eine lange Lebensdauer des Kontaktes sicherzustellen, ist es erforderlich, während der Alkylierung kontinuierlich Phosphorsäure und/oder Phosphorsäurealkylester zuzuführen; ein Teil dieser Phosphorverbindungen wird jedoch stets ausgetragen und muß vom Reaktionsprodukt abgetrennt werden.

Es bestand daher die Aufgabe, Katalysatoren zur Verfügung zu stellen, mit Hilfe derer verschiedenartige Anilin-Verbindungen in der Gasphase am N-Atom alkyliert werden können und die die vorgenannten Nachteile nicht aufweisen. Die Katalysatoren sollten sich durch einfache Verfügbarkeit, lange Standzeiten und hohe Aktivitäten auszeichnen und hohe Umsätze mit guter Selektivität gewährleisten.

Es wurde nun überraschend gefunden, daß bestimmte Aluminiumoxide, die weiter unten näher beschrieben werden, vorteilhaftere Katalysatoren für die N-Alkylierung von Anilinen mit Alkoholen darstellen als die bisher bekannten. Das neue Verfahren gewährleistet die geforderten hohen Ausbeuten und Umsätze, es tritt praktisch keine Kernalkylierung auf, und die verwendeten Katalysatoren weisen sehr hohe Standzeiten auf.

Die Erfindung betrifft daher ein Verfahren zur N-Alkylierung von Anilinen oder N-Alkyl-anilinen mit niederen Alkoholen oder den zugehörigen Dialkylethern bei erhöhter Temperatur in der Gasphase an $Al_2O_3$-Katalysatoren, das dadurch gekennzeichnet ist, daß als Katalysator ein $\gamma$-$Al_2O_3$ eingesetzt wird, das durch Calcinieren eines Aluminiumoxidhydroxid-Hydrats bei 500°C bis 1000°C hergestellt wird, eine BET-Oberfläche von 50 bis 400 $m^2/g$, bevorzugt 100 bis 350, besonders bevorzugt 150 bis 300 $m^2/g$, und eine Oberflächenacidität von 5 bis 600 mmol $H^+$/kg, bevorzugt 10 bis 400 mmol $H^+$/kg, besonders bevorzugt 20-300 mmol $H^\oplus$/kg besitzt.

Aluminiumoxidhydroxid-Hydrate für die Herstellung der erfindungsgemäß einzusetzenden Katalysatoren können durch Hydrolyse von Aluminiumsalzen, Aluminiumalkoholaten, Aluminiumphenolaten oder anderen geeigneten hydrolisierbaren Aluminiumverbindungen hergestellt werden. Die Hydrolyse kann in bekannter Weise durch verdünnte Säuren oder verdünnte Alkalien, in vielen Fällen auch durch Wasser allein vorgenommen werden. Die Durchführung einer solchen Hydrolyse ist dem Fachmann grundsätzlich bekannt. Es hat sich herausgestellt, daß sich in besonderer Weise Hydrolysate von Aluminiumalkoholaten für die Herstellung erfindungsgemäß einsetzbarer Katalysatoren eignen. In besonders bevorzugter Weise stammen solche Aluminiumalkoholate aus der Oxidation von Trialkylaluminiumverbindungen, beispielsweise aus dem Alfol®-Prozeß (Ullmanns Enzyklopädie der technischen Chemie, 4. Aufl. Bd. 7, S. 208 ff und 5. Aufl., Bd. A 10, S. 283 ff).

Bei diesem Prozeß zur Herstellung von Alkoholen durch Oxidation aluminiumorganischer Verbindungen und Hydrolyse der entstehenden Alkoxyverbindungen mit Wasser erhält man ein Aluminiumoxidhydroxid-Hydrat von besonderer Qualität, das nur sehr geringe Mengen an Verunreinigungen enthält.

Aluminumoxidhydroxid-Hydrate werden zur Herstellung von Granulaten, Extrudaten, Kugeln oder mit Wasser dispergierbaren Pulvern mit Mineralsäuren und/oder organischen Säuren wie z.B. $HNO_3$, HBr, HCl, $CH_3COOH$ u.a. peptisiert. Bei der Herstellung der Formlinge wird die Säure/Aluminiumoxidhydroxid-Mischung vor der Verarbeitung in einem Kneter einige Minuten durchgeknetet. Die feuchten Formkörper werden anschließend bei 110°C getrocknet und drei Stunden bei 500°C bis 1000°C calciniert, wobei die $\gamma$-Modifikation des $Al_2O_3$ entsteht.

Die hierbei entstehenden Aluminumoxide weisen BET-Oberflächen von 50 bis 400 $m^2/g$ auf.

Durch die Peptisation mit der Säure erhält man im Granulat in Abhängigkeit von der eingesetzten Säuremenge eine Oberflächenacidität von 5 bis 600 mmol $H^+/kg$.

Eine weitere Ausführungsform zur Herstellung der $Al_2O_3$-Granulate für das erfindungsgemäße Verfahren besteht darin, daß als Ausgangsmaterial ein vorpeptisiertes, mit Wasser dispergierbares Aluminumoxidhydroxid eingesetzt wird. Dieses Pulver wird mit Wasser auf die Granulierfeuchte eingestellt und mit geeigneten Verformungsgeräten zu Kugeln, Extrudaten und Granulaten verarbeitet. Die Calcinierung erfolgt ebenfalls bei 500°C bis 1000°C innerhalb von drei Stunden.

Die Granulate weisen wiederum eine BET-Oberfläche von 50 bis 400 $m^2/g$ auf.

Die Oberfächenacidität dieser Granulate liegt im genannten Bereich, bevorzugt bei 20 bis 300 mmol $H^+/kg$.

Es war nicht vorhersehbar, daß solche speziellen $\gamma$-$Al_2O_3$-Katalysatoren mit den beschriebenen Eigenschaften sich in Bezug auf die Standzeit und die Effektivität als so günstig herausstellen, daß sie im Gegensatz zu den aus der Literatur bekannten $Al_2O_3$-Katalysatoren für eine industrielle Anwendung geeignet sind.

Die Oberflächenacidität des erhaltenen $\gamma$-$Al_2O_3$ und damit dessen Eignung für das erfindungsgemäße Verfahren wird am einfachsten durch direkte Titration dieses $\gamma$-$Al_2O_3$ in wäßriger Suspension bestimmt.

Das erfindungsgemäße Verfahren zur N-Alkylierung von Anilinen oder N-monoalkylsubstituierten Anilinen wird bei 200 bis 400°C, bevorzugt bei 250 bis 350°C, besonders bevorzugt bei 260 bis 320°C, in der Gasphase durchgeführt. Der Katalysator kann hierbei sowohl im Festbett als auch im Fließbett angeordnet sein. Das erfindungsgemäße Verfahren wird bei Normaldruck durchgeführt; es kann jedoch auch bei wenig erniedrigtem oder wenig erhöhtem Druck durchgeführt werden. Als Druckbereich sei daher ein solcher von 0,7 bis 2 bar, genannt.

Zur Durchführung kann man beispielsweise ein Gemisch des am N-Atom zu alkylierenden Anilins und des Alkylierungsmittels Alkohol bzw. Ether verdampfen und das verdampfte Gemisch über den Kontakt leiten. Es ist möglich, ein Inertgas, wie Stickstoff, Helium, Wasserdampf, Wasserstoff oder Argon als Trägergas dem Einsatzgemisch zuzusetzen.

Die Katalysatorbelastung (Liquid Hourly Space Velocity = LHSV) kann im Bereich von 0,1 bis 4,0 l/l/h, bevorzugt von 0,3 bis 2,0 l/l/h, variiert werden. Die LHSV ist hierbei als das Verhältnis des Volumens des Anilin/Alkohol- bzw. Anilin/Dialkylether-Gemisches pro Katalysatorvolumen pro Stunde definiert.

Der Katalysator wird zweckmäßigerweise in Form eines Granulats eingesetzt, wenn im Festbett gearbeitet wird; für das Arbeiten im Fließbett eignet sich in bekannter Weise eine feinkörnige Verteilung.

Das Reaktionsgemisch wird am Reaktorausgang kondensiert und beispielsweise destillativ aufgetrennt. Nicht umgesetzte Ausgangsstoffe können in bekannter Weise in die Reaktion zurückgeführt werden. Für den Fall, daß N-monoalkylierte oder N,N-dialkylierte Aniline aus nicht am N-Atom substituierten Anilinen hergestellt werden sollen, werden die am N-Atom monosubstituierten und disubstituierten Aniline als Reaktionsprodukte ebenfalls aufgetrennt, wobei das nicht gewünschte Reaktionsprodukt ebenfalls in die Reaktion zurückgeführt wird.

In das erfindungsgemäße Verfahren werden Aniline oder N-Alkylaniline der Formel

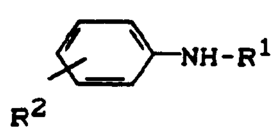

(I)

eingesetzt, worin

$R^1$ Wasserstoff oder geradkettiges oder verzweigtes $C_1$-$C_{10}$-Alkyl und

$R^2$ Wasserstoff, geradkettiges oder verzweigtes $C_1$-$C_{10}$-Alkyl, $C_6$-$C_{12}$-Aryl, $C_1$-$C_4$-Alkoxy, Halogen, Cyano oder Nitro bedeuten.

Geradkettiges oder verzweigtes $C_1$-$C_{10}$-Alkyl ist beispielsweise Methyl, Ethyl, Propyl, Isopropyl, Butyl, Isobutyl, tert.-Butyl, die isomeren Hexyle, Octyle oder Decyle. In bevorzugter Weise seien die genannten $C_1$-$C_4$-Alkylreste genannt.

$C_6$-$C_{12}$-Aryl ist beispielsweise Phenyl, Naphthyl oder Biphenylyl, bevorzugt Phenyl. $C_1$-$C_4$-Alkoxy ist bei-

spielsweise Methoxy, Ethoxy, Propoxy, Isopropoxy, Butoxy oder Isobutoxy, bevorzugt Methoxy oder Ethoxy. Halogen ist beispielsweise Fluor, Chlor oder Brom, bevorzugt Chlor.

Als geeignete Amine seien beispielsweise aufgezählt: Anilin, o-, m-, p-Toluidin, o-, m-, p-Chloranilin, 2,4-, 2,6-, 3,4- und 3,5-Xylidin, o-, m-, p-Ethylanilin, o-, m-, p-Isopropylanilin und andere; insbesondere seien Anilin und die genannten Toluidine hervorgehoben.

Als zur Alkylierung geeignete Alkohole seien die dem Fachmann geläufigen niederen Alkohole, beispielsweise solche mit 1 bis 4 C-Atomen genannt, wie Methanol, Ethanol, Propanol, Isopropanol oder die verschiedenen Butanole.

Bevorzugt werden Methanol oder Ethanol eingesetzt. Die zugehörigen Dialkylether $(C_1\text{-}C_4\text{-Alkyl})_2O$ sind dem Fachmann gleichfalls bekannt; es handelt sich um Dimethylether, Diethylether, Dipropylether, Diisopropylether, Dibutylether und die denkbaren gemischten Ether. In bevorzugter Weise wird Dimethylether oder Diethylether eingesetzt. Beim Einsatz eines Ethers wird dieser wie 2 Mol des korrespondierenden Alkohols gerechnet.

Je nachdem, ob der Rest $R^1$ in der Formel (I) Wasserstoff oder einen Alkylrest bedeutet, läuft das erfindungsgemäße Verfahren nach einer der beiden folgenden Formelgleichungen ab:

a)

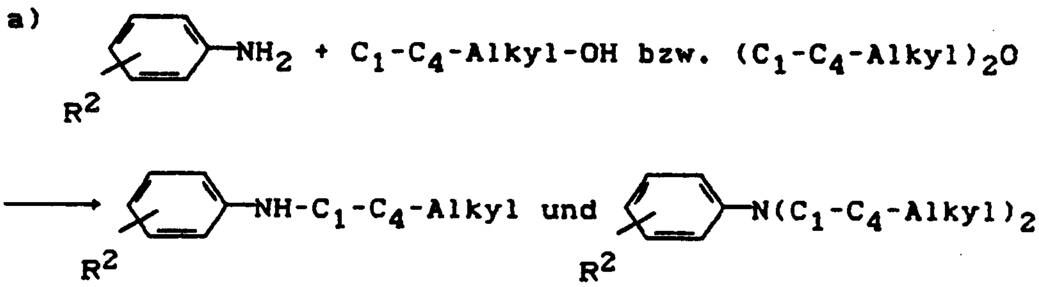


bzw.

b)

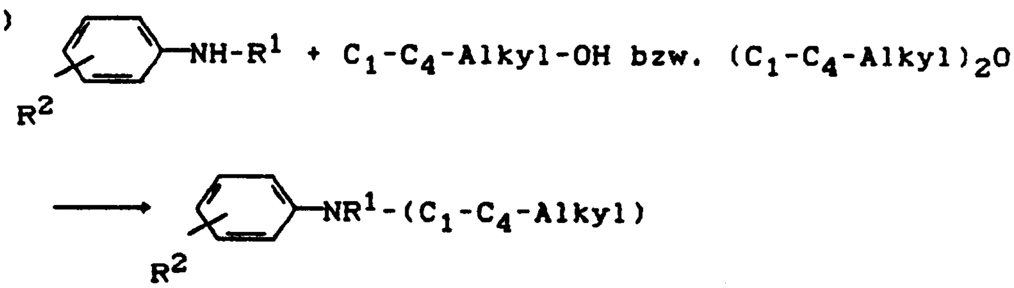


Bei der Umsetzung gemäß a) wird ein am N-Atom nicht substituiertes Anilin durch einen Alkohol oder einen Dialkylether in ein Gemisch des am N-Atom monosubstituierten und des disubstituierten Anilins umgesetzt. Gemäß Umsetzung b) wird ein am N-Atom bereits einfach alkylsubstituiertes Anilin zu einem am N-Atom dialkylierten Anilin umgesetzt. Hierbei kann je nach dem bereits vorhandenen Substituenten $R^1$ auch ein gemischt alkyliertes Anilin erhalten werden.

Das Molverhältnis des Alkylierungsmittels Alkohol bzw. Ether zum Anilin beträgt 0,2 bis 10 Mol Alkohol bzw. 0,1 bis 5 Mol Ether pro Mol Anilin bzw. N-Alkyl-anilin bevorzugt 0,4 bis 6, bzw. 0,2 bis 3, besonders bevorzugt 0,5 bis 4 bzw. 0,25 bis 2. Die Wahl des Molverhältnisses gestattet in einer dem Fachmann grundsätzlich bekannten Weise, das Verhältnis von N-Monoalkyl- zu N,N-Dialkylanilin im Produkt zu steuern, falls von einem am N-Atom noch nicht substituierten Anilin ausgegangen wird. Ein Überschuß an Alkohol gegenüber Anilin bewirkt eine Verschiebung der Selektivität zugunsten des N,N-Dialkylanilins. Ein kleines Verhältnis von Anilin zu Alkohol ermöglicht eine hohe Selektivität für N-Monoalkylanilin.

Besteht ein hoher Bedarf an Monoalkylverbindungen, so kann ein gegebenenfalls erzeugter Überschuß an Dialkylanilin in die Reaktion zurückgeführt und in Monoalkylanilin übergeführt werden. Analoges gilt für den Fall, daß N,N-Dialkylverbindungen in großem Maß verlangt werden, dann lassen sich die Monoalkylaniline recyclisieren und höher alkylieren.

## Beispiele

### Katalysator-Zubereitungsbeispiel A

Zu 20 kg Aluminiumoxidhydroxid-Hydrat (z.B. Pural SCF®, Fa. Condea) in einem Kneter wurden 12 kg 1,5 Gew.-% $HNO_3$ über 7 Min. in einem konstanten Strom zugegeben. Unter weiterem Kneten gab man anschließend weitere 7 kg 1,5 Gew.-% $HNO_3$ innerhalb von 8 Min. zu. Nach der Verformung mit entsprechenden Granulierapparaten wurden die Granulate bei 110°C getrocknet und drei Stunden bei 550°C calciniert. Das so erhaltene Granulat wies eine Oberflächenacidität von 30 mmol $H^+$/kg auf.

### Katalysator-Zubereitungsbeispiel B

10 kg eines vorpeptisierten Aluminiumoxidhydroxid-Hydrats (z.B. Disperal spezial 10/I®, Fa. Condea) wurden mit Wasser granulierfeucht eingestellt und ca 15 Min. in einem Kneter geknetet. Anschließend wurde die feuchte Mischung zu Formlingen verarbeitet, getrocknet und drei Stunden bei 550°C calciniert. Das so erhaltene Granulat wies eine Oberflächenacidität von 90 mmol $H^+$/kg auf.

### Katalysator-Zubereitungsbeispiel C (Vergleich)

500 g eines handelsüblichen $\gamma$-$Al_2O_3$-Granulats (Alumine Aktivee A, Fa. Rhone Poulenc) wurden mit 0,5 ml einer 1N $HNO_3$-Lösung getränkt, getrocknet und bei 550°C aktiviert. Das Material hatte eine Oberflächenacidität von < 5 mmol $H^+$/kg.

### Aciditätsbestimmung

Zur Bestimmung der Oberflächenacidität wurde 1 g eines zu Pulver zermahlenen Aluminiumoxid-Granulats in 40 ml Wasser suspendiert. Die Suspension wurde bei einer Tropfengeschwindigkeit von 0,1 ml/Min. mit 0,1 N NaOH potentiometrisch titriert.

Die Aufmahlung der Granulat-Proben erfolgte in einem Mikro-Dismembrator (Fa. Braun Melsungen AG). In den Unterteil einer Mahlvestole wurden 5 ml des Granulats und eine Wolframcarbid-Kugel mit einem Durchmesser von 9 mm gegeben. Die Vestole wurde mit dem Oberteil verschlossen und in den Schüttelbehälter aus PTFE gegeben. Der Schüttelbehälter wurde in die Halterung des Dismembrators eingespannt und die Schwingamplitude auf ca. 10 mm Ausschlag eingestellt. Nach 6 bis 8 Min. war die Mahlung beendet. Das feinteilige Pulver konnte dann für die obige Aciditätsbestimmung eingesetzt werden.

### Beispiele 1-4

In einem senkrecht stehenden Reaktionsrohr von 40 cm Länge und einem Durchmesser von 30 mm wurden 25 ml des erfindungsgemäß zu verwendenden Aluminiumoxids nach Beispiel A oder B, das eine mittlere Korngröße von 1 bis 2 mm besaß, eingebracht. Mittels eines organischen Wärmeträgers ließ sich der Reaktor thermostatisieren. Die Tempertur in der Katalysatorschüttung konnte mittels eines beweglichen Thermoelementes gemessen werden und betrug 300°C. Über eine Dosiervorrichtung wurde ein Gemisch von Anilin und Alkohol im Molverhältnis 1:1 in einen Verdampfer eindosiert, dort in die Gasphase übergeführt und über den Kontakt geleitet. Die Dosiergeschwindigkeit betrug 20 ml/h flüssiges Gemisch; dies entsprach einer Katalysatorbelastung (LHSV) von 0,8 l/l/h. Das Reaktionsprodukt wurde auskondensiert und gaschromatograhisch analysiert.

Umsatz und Selektivität sind in Tabelle 1 zusammengestellt.

### Vergleichsbeispiele 1 und 3

In der Vorrichtung aus Beispiel 1 bis 4 wurde ein handelsübliches $\gamma$-Aluminiumoxid, das nicht über die Hydrolyse von Aluminiumalkoholaten oder -salzen hergestellt worden war, sondern beispielsweise aus dem Bayer-Prozeß (Ullmann, 4. Aufl., Bd. 7, S. 305 ff und 5. Aufl., Bd. A1, S. 566 ff) stammt und eine geringe Oberflächenacidität besitzt, unter identischen Bedingungen als Katalysator eingesetzt. Die Vergleichsexperimente sind ebenfalls in Tabelle 1 aufgeführt.

### Vergleichsbeispiele 2 und 4

In der Vorrichtung aus Beispiel 1 bis 4 wurde der nach Zubereitungsbeispiele C erhaltene Katalysator unter

identischen Bedingungen eingesetzt. Die Ergebnisse sind ebenfalls in Tabelle 1 aufgenommen.

Entsprechend den Beispielen 1 bis 4 sind die erfindungsgemäß zu verwendenden γ-Aluminiumoxide gute Katalysatoren zur N-Alkylierung von Anilinen mit Alkoholen in der Gasphase bei Normaldruck. Aus Tabelle 1 wird klar ersichtlich, daß γ-Aluminiumoxide, die nicht erfindungsgemäß hergestellt wurden, zwar ebenfalls katalytisch aktiv sind, jedoch einen unbefriedigenden Umsatz zeigen, obwohl sie eine größere BET-Oberfläche als die erfindungsgemäß zu verwendenden Aluminumoxide besitzen. Trotz geringerem Umsatz war der Anteil an nicht erwünschten Kernalkylierungsprodukten größer als bei den erfindungsgemäß zu verwendenden Aluminiumoxiden. Durch eine nachträgliche Säurebehandlung kann eine geringe Erhöhung der Aktivität der nicht erfindungsgemäß hergestellten Aluminiumoxid-Materialien bewirkt werden. Der Umsatz bleibt jedoch weiterhin deutlich unterhalb des mit den erfindungsgemäß zu verwendenden Katalysatoren erreichbaren Niveaus. Auf die Methylierung von Anilin hat die nachträgliche Säurebehandlung überhaupt keinen Effekt.

**Tabelle 1:** N-Alkylierung von Anilin mit Alkoholen

| Beispiel | Kat. | Katalysator-Acidität (mmol $H^+$/kg) | BET-Oberfl. ($m^2$/g) | Alkohol | Umsatz Anilin (%) | Selektivität (%) N-Alkyl-anilin | Selektivität (%) N,N-Dialkyl-anilin | Selektivität (%) Kern-alkyl |
|---|---|---|---|---|---|---|---|---|
| 1 | A | 30 | 230 | EtOH | 68 | 89,4 | 9,7 | 0,9 |
| 2 | B | 90 | 160 | EtOH | 66 | 89,8 | 9,4 | 0,8 |
| Vgl. 1 | 1) | <5 | 280 | EtOH | 49 | 92,2 | 6,4 | 1,4 |
| Vgl. 2 | C | <5 | 280 | EtOH | 58 | 91,0 | 7,7 | 1,3 |
| 3 | A | 30 | 230 | MeOH | 72 | 67,7 | 32,0 | 0,3 |
| 4 | B | 90 | 160 | MeOH | 74 | 61,5 | 38,1 | 0,4 |
| Vgl. 3 | 1) | <5 | 280 | MeOH | 63 | 70,6 | 28,8 | 0,6 |
| Vgl. 4 | C | <5 | 280 | MeOH | 62 | 69,4 | 28,5 | 2,1 |

1) handelsübliches γ-Aluminiumoxid-Granulat Alumine Activee A der Fa. Rhone-Poulenc

Die folgenden Beispiele sollen die Flexibilität des Verfahrens hinsichtlich Substratvariabilität und Selektivität sowie das Standzeitverhalten der Katalysatoren verdeutlichen.

Beispiele 5 bis 8

In einem senkrecht stehenden Reaktionsrohr von 150 cm Länge und einem Innendurchmesser von 32 mm wurden 1 l eines γ-Aluminiumoxids nach Beispiel A oder B, das eine mittlere Korngröße von 2 bis 3 mm besaß, eingebracht. Mittels eines organischen Wärmeträgers ließ sich der Reaktor thermostatisieren. Die Temperatur über die gesamte Katalysatorschüttung konnte mittels eines beweglichen Thermoelementes gemessen werden. Über eine Pumpe wurde die flüssige Reaktionsmischung dem Verdampfer zugeführt, in die Gasphase übergeführt und über den Kontakt geleitet. Das Reaktionsprdukt wurde kondensiert und gaschromatographisch analysiert. Die genauen Versuchsbedingungen und die Ergebnisse der Beispiele 5 bis 8 sind in Tabelle 2 zusammengefaßt.

Nach 4000 Stunden Katalysatorbelastung waren Umsatz und Selektivität unverändert.

Die Beispiele zeigen, daß eine sehr flexible Steuerung der Produktselektivität möglich ist. Die Parameter, die für den Grad der Alkgierung verantwortich sind, sind die Katalysatorbelastung (LHSV), das Molverhältnis von Anilin zu Alkohol sowie die Reaktionstemperatur. Ein kleines Verhältnis von Anilin zu Alkohol ermöglicht bei hoher Katalysatorbelastung eine sehr selektive Monoalkylierung. Bei Umsätzen von 45 % bzw. 49 % werden Selektivitäten von 87 % bis 95 % Monoalkylanilin erzielt (Beispiele 6, 8). Mit anderen als den erfindungsgemäß zu verwendenden Aluminumoxiden werden unter analogen Reaktionsbedingungen deutlich geringere Umsätze erzielt. Ein Überschuß an Alkohol, eine geringe Katalysatorbelastung sowie eine hohe Temperatur führen zu hohem Anilin-Umsatz und hoher Dialkylselektivität, die über 90 % erreichen kann (Beispiel 7).

Beispiel 9

Entsprechend Beispiel 2 wurde ein Gemisch aus Anilin und Diethylether im Molverhältnis 1:0,5 bei 300°C und einer Fließgeschwindigkeit von 0,8 l/l/h umgesetzt. Der Umsatz an Anilin betrug 69 %. Die Selektivitäten betrugen 87,2 % für N-Ethylanilin, 11,8 % für N,N-Diethylanilin und 1,0 % für die Summe der kernalkyierten Nebenprodukte.

**Tabelle 2:** N-Alkylierung von aromatischen Aminen

| Bsp. | Kat. | Katalysator Acidität (mmol $H^+$/kg) | BET-Oberfl. ($m^2$/g) | Anilin | Alk.-mittel | Anilin: Alk.mittel | LHSV (l/l/h) | T (°C) | Umsatz Anilin (%) | Selektivität (%) N-Mono- | N,N-Di- | Kern-alkyl |
|---|---|---|---|---|---|---|---|---|---|---|---|---|
| 5 | A | 30 | 230 | Anilin | MeOH | 1:1 | 0,5 | 310 | 75 | 63,8 | 35,9 | 0,3 |
| 6 | B | 90 | 160 | Anilin | MeOH | 1:1 | 1,5 | 270 | 45 | 86,8 | 13,1 | 0,1 |
| 7 | B | 90 | 160 | Anilin | MeOH | 1:3 | 0,3 | 310 | 99 | 8,5 | 91,4 | 0,1 |
| 8 | B | 90 | 160 | m-Toluidin | EtOH | 1:1 | 1,5 | 300 | 49 | 94,8 | 4,5 | 0,7 |

## Patentansprüche

1. Verfahren zur N-Alkylierung von Anilinen oder N-Alkyl-anilinen mit niederen Alkoholen oder den zugehörigen Dialkylethern bei erhöhter Temperatur in der Gasphase an $Al_2O_3$-Katalysatoren, dadurch gekennzeichnet, daß als Katalysator ein $\gamma$-$Al_2O_3$ eingesetzt wird, das durch Entwässerung eines Aluminiumoxidhydroxid-Hydrats hergestellt wird, eine BET-Oberfläche von 50 bis 400 $m^2/g$, bevorzugt 100 bis 350 $m^2/g$, besonders bevorzugt 150 bis 300 $m^2/g$, und eine Oberflächenacidität von 5 bis 600 mmol $H^+/kg$, bevorzugt 10 bis 400 mmol $H^+/kg$, besonders bevorzugt 20-300 mmol $H^+/kg$, besitzt.

2. Verfahren nach Anspruch 1, dadurch gekennzeichnet, daß das $\gamma$-$Al_2O_3$ durch Entwässern eines Hydrolysats von Aluminiumalkoholaten hergestellt wird.

3. Verfahren nach Anspruch 2, dadurch gekennzeichnet, daß ein Aluminiumalkoholat aus der Oxidation von Trialkylaluminimverbindungen eingesetzt wird.

4. Verfahren nach Anspruch 1, dadurch gekennzeichnet, daß bei 200 bis 400°C, bevorzugt bei 250 bis 350°C, besonders bevorzugt bei 260 bis 320°C, gearbeitet wird.

5. Verfahren nach Anspruch 1, dadurch gekennzeichnet, daß bei einem Druck von 0,7 bis 2 bar gearbeitet wird.

6. Verfahren nach Anspruch 1, dadurch gekennzeichnet, daß ein Anilin oder N-Alkyl-anilin der Formel

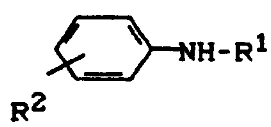

eingesetzt wird, worin

$R^1$ Wasserstoff oder geradkettiges oder verzweigtes $C_1$-$C_{10}$-Alkyl und

$R^2$ Wasserstoff, geradkettiges oder verzweigtes $C_1$-$C_{10}$-Alkyl, $C_6$-$C_{12}$-Aryl, $C_1$-$C_4$-Alkoxy, Halogen, Cyano oder Nitro bedeuten.

7. Verfahren nach Anspruch 1, dadurch gekennzeichnet, daß ein $C_1$-$C_4$-Alkohol oder der zugehörige Di-$C_1$-$C_4$-Alkyl-ether in einer Menge von 0,2 bis 10 Mol Alkohol bzw. 0,1 bis 5 Mol Ether pro Mol Anilin bzw. N-Alkyl-anilin eingesetzt wird.

8. Verfahren nach Anspruch 1, dadurch gekennzeichnet, daß bei einer Katalysatorbelastung (Liquid Hourly Space Velocity = LHSV) von 0,1 bis 4,0 l/l/h, bevorzugt 0,3 bis 2,0 l/l/h, verstanden als Volumen des Anilin/Alkohol- bzw. Anilin/Ether-Gemisches pro Katalysatorvolumen, gearbeitet wird.

## Claims

1. Process for the N-alkylation of anilines or N-alkyl-anilines with lower alcohols or the associated dialkyl ethers at elevated temperature in the gas phase on $Al_2O_3$ catalysts, characterized in that the catalyst used is a y-$Al_2O_3$ that is prepared by dehydration of an aluminium oxide/hydroxide hydrate and has a BET surface area of from 50 to 400 $m^2/g$, preferentially 100 to 350 $m^2/g$, particularly preferentially 150 to 300 $m^2/g$, and a surface acidity of 5 to 600 mmol $H^+/kg$, preferentially 10 to 400 mmol $H^+/kg$, particularly preferentially 20-300 mmol $H^+/kg$.

2. Process according to Claim 1, characterized in that the $\gamma$-$Al_2O_3$ is prepared by dehydration of a hydrolysate of aluminium alcoholates.

3. Process according to Claim 2, characterized in that an aluminium alcoholate from the oxidation of trialkylaluminium compounds is used.

4. Process according to Claim 1, characterized in that the process is carried out at 200 to 400°C, preferentially at 250 to 350°C, particularly preferentially at 260 to 320°C.

5. Process according to Claim 1, characterized in that the process is carried out at a pressure of 0.7 to 2 bars.

6. Process according to Claim 1, characterized in that an aniline or N-alkyl-aniline of the formula is used, in which
   - $R^1$ denotes hydrogen or straight-chain or branched $C_1$-$C_{10}$-alkyl and
   - $R^2$ denotes hydrogen, straight-chain or branched $C_1$-$C_{10}$-alkyl, $C_6$-$C_{12}$-aryl, $C_1$-$C_4$-alkoxy, halogen, cyano or nitro.

7. Process according to Claim 1, characterized in that a $C_1$-$C_4$ alcohol or the associated di-$C_1$-$C_4$-alkyl ether is used in an amount of from 0.2 to 10 moles of alcohol or 0.1 to 5 moles of ether per mole of aniline or N-alkyl-aniline.

8. Process according to Claim 1, characterized in that the process is carried out at a catalyst loading (Liquid Hourly Space Velocity = LHSV) of 0.1 to 4,0 l/l/h, preferably 0.3 to 2.0 l/l/h, understood as the volume of the aniline/alcohol or aniline/ ether mixture per catalyst volume.

## Revendications

1. Procédé pour la N-alkylation d'anilines ou d'anilines N-alkylées, avec des alcools inférieurs ou avec les éthers dialkyliques correspondants, à température élevée, en phase gazeuse, sur des catalyseurs de $Al_2O_3$, qui se caractérise par le fait qu'on met en oeuvre, comme catalyseur, un $\gamma$-$Al_2O_3$ que l'on prépare par déshydratation d'un hydrate d'oxyde-hydroxyde d'aluminium, qui possède une surface BET de 50 à 400 $m^2/g$, de préférence de 100 à 350, de manière particulièrement préférée de 150 à 300 $m^2/g$, ainsi qu'une acidité superficielle de 5 à 600 mmoles $H^+/kg$, de préférence de 10 à 400 mmoles $H^+/kg$, de manière particulièrement préférée de 20 à 300 mmoles $H^+/kg$.

2. Procédé selon la revendication 1, caractérisé en ce qu'on prépare le $\gamma$-$Al_2O_3$ par déshydratation d'un hydrolysat d'alcoolates d'aluminium.

3. Procédé selon la revendication 2, caractérisé en ce qu'on met en oeuvre un alcoolate d'aluminium à partir de l'oxydation de composés de trialkylaluminium.

4. Procédé selon la revendication 1, caractérisé en ce qu'on travaille à une température de 200 à 400°C, de préférence de 250 à 350°C, de manière particulièrement préférée de 260 à 320°C.

5. Procédé selon la revendication 1, caractérisé en ce qu'on travaille sous une pression de 0,7 à 2 bar.

6. Procédé selon la revendication 1, caractérisé en ce qu'on met en oeuvre une aniline ou une N-alkylaniline répondant à la formule

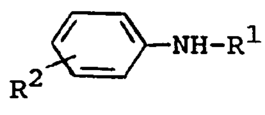

   dans laquelle
   - $R^1$ représente un atome d'hydrogène ou un groupe alkyle en $C_1$-$C_{10}$ à chaîne droite ou ramifiée et
   - $R^2$ représente un atome d'hydrogène, un groupe alkyle en $C_1$-$C_{10}$ à chaîne droite ou ramifiée, un groupe aryle en $C_6$-$C_{12}$, un groupe alcoxy en $C_1$-$C_4$, un atome d'halogène, un groupe cyano ou un groupe nitro.

7. Procédé selon la revendication 1, caractérisé en ce qu'on met en oeuvre un alcool en $C_1$-$C_4$ ou l'éther dialkylique en $C_1$-$C_4$ correspondant en une quantité de 0,2 à 10 moles d'alcool ou de 0,1 à 5 moles d'éther par mole d'aniline ou de N-alkylaniline.

**8.** Procédé selon la revendication 1, caractérisé en ce qu'on travaille avec une charge de catalyseur (Liquid Hourly Space Velocity = vitesse spatiale horaire de liquide) de 0,1 à 4,0 l/l/h, de préférence de 0,3 à 2,0 l/l/h, représentant le volume du mélange aniline/alcool ou aniline/éther par volume de catalyseur.